# EUROPEAN PATENT SPECIFICATION

(11) **EP 2 092 342 B1**
(45) Date of publication and mention of the grant of the patent: **21.07.2010**
(21) Application number: 06842803.6
(22) Date of filing: 22.12.2006
(51) Int. Cl.: G01N 33/68, G01N 33/50

(54) **A METHOD FOR MEASURING PLASMA LEVELS OF PENTRAXIN PTX3**
VERFAHREN ZUR MESSUNG VON PLASMASPIEGELN VON PENTRAXIN PTX3
PROCÉDÉ DE MESURE DE LA CONCENTRATION PLASMIQUE DE LA PENTRAXINE PTX3

(43) Date of publication of application: 26.08.2009
(73) Proprietor: Humanitas Mirasole S.p.A., 20089 Rozzano (Milano) (IT)
(72) Inventor: MANTOVANI, Alberto, I-20089 ROZZANO (Milano) (IT); PERI, Giuseppe, I-20089 ROZZANO (Milano) (IT); PASQUALINI, Fabio, I-20089 ROZZANO, (Milano) (IT)
(74) Representative: Long, Giorgio
(86) International application number: PCT/IT2006/000872
(87) International publication number: WO 2008/078344

(56) References cited:
- WO-A-94/24555
- GB-A- 1 325 043
- US-A1- 2004 137 544
- INOUE KENJI ET AL: "Establishment of a high sensitivity plasma assay for human pentraxin3 as a marker for unstable angina pectoris." ARTERIOSCLEROSIS, THROMBOSIS, AND VASCULAR BIOLOGY JAN 2007, vol. 27, no. 1, January 2007 (2007-01), pages 161-167, XP009084262 ISSN: 1524-4636
- LATINI ROBERTO ET AL: "Prognostic significance of the long pentraxin PTX3 in acute myocardial infarction." CIRCULATION 19 OCT 2004, vol. 110, no. 16, 19 October 2004 (2004-10-19), pages 2349-2354, XP002435282 ISSN: 1524-4539

## Description

The present invention relates to a method for measuring PTX3 in a biological fluid, particularly in human or animal plasma.

Long pentraxin PTX3 is a protein belonging to the pentraxin family and is a biological marker useful for the early diagnosis of risk of death in individuals affected by cardiovascular disorders. The pentraxins, so called due to their ability to form pentamers, are a group of proteins comprising C-reactive protein (CRP) and serum amyloid P (SAP), produced by the liver in response to inflammatory mediators. CRP and SAP are considered to be the most typical acute phase proteins, and the circulating levels thereof are elevated during inflammation, infection and tissue damage. PTX3 is a novel member of this family and has been cloned from endothelial cells stimulated with interleukin-1. The molecule is constituted by two structural domains, one N-terminal with no homology to any known proteins, and one C-terminal, similar to those of the short pentraxins such as CRP and SAP. Contrary to the situation with the short pentraxins, where the main site of production is the liver, PTX3 is produced by various cell types, particularly endothelial cells, monocytes-macrophages and dendritic cells in response to proinflammatory proteins, such as IL-1 and TNF and bacterial products. Previous studies have shown that circulating levels of PTX3 are raised in patients affected by acute or chronic inflammatory disorders such as sepsis and myocardial infarction. In particular, the authors of the present invention have conducted a trial on 37 patients with myocardial infarction and have observed that levels of PTX3 reach a peak of 6.94 ng/ml ± 11.26 7.5 hours after admission to the coronary unit, while the peak of CRP is observed 24 hours after admission.Recently, in a much wider study (724 patients with myocardial infarction) it has been shown that raised levels of PTX3 on the first day following infarction are associated with increased risk of mortality or the onset of heart failure in the first three months following the event. PTX3 binds to both molecules present in the blood, such as CRP, and Fibroblast Growth Factor-2.

These latest observations pertaining to the ability of PTX3 to bind to molecules present in the blood might be the cause of the difficulties associated with measuring circulating PTX3 levels encountered to date. Indeed, the US patent publication US 2004137544 describes a method for determining plasma levels of PTX3, which nevertheless has a significant drawback. Indeed, applying said method, the values for serum PTX3 are on average half the values measured in EDTA-plasma, and said difference is not always constant, but varies from one subject to another. Furthermore, while for serum, the values are reproducible over time and after one or two freeze-thawing cycles, in EDTA-plasma or in citrate-plasma, the values normally tend to increase with passing time and after repeated freeze-thawing. It has also been observed that in both serum and EDTA-plasma or citrate-plasma there are factors present which inhibit measurement, in that with the addition of a standard curve of PTX3 in the aforementioned elements, it is not possible to obtain the same optical density (O.D.) values which are obtained using the same concentrations of PTX3 in a solution of RPMI 1640 medium + 2% bovine serum albumin (BSA) or PBS + 2% BSA.

Hence, there is a need to provide a method for determining plasma levels of PTX3 which allows measuring all the PTX3 present in the sample under test, thus avoiding the measurement of false negatives.

Said need is satisfied by a method for measuring long pentraxin PTX3 in bodily fluids as defined in the appended claims, the definitions of which form an integral part of the present description.

### Brief description of the figures

Figure 1 is a histogram showing PTX3 levels in EDTA-plasma with and without the addition of an agglutinating agent (polybrene^{®});
Figure 2 is a histogram showing PTX3 levels in EDTA-plasma supplemented with an agglutinating agent (polybrene^{®}) measured immediately and after 24 hours at 4°C;
Figure 3 is a graph representing standard curves of PTX3 in EDTA-plasma with and without an agglutinating agent (polybrene^{®});
Figure 4 is a histogram showing PTX3 levels in EDTA-plasma supplemented with an agglutinating agent (polybrene^{®}) after 1, 2 and 3 freeze-thawing cycles respectively.

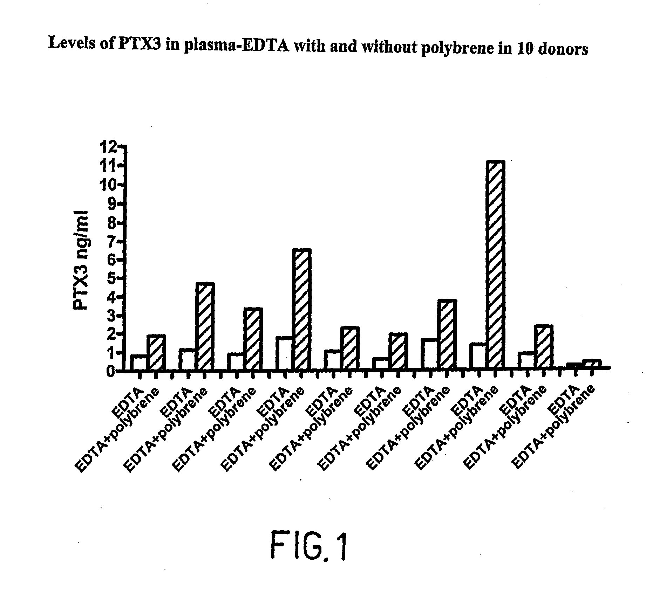

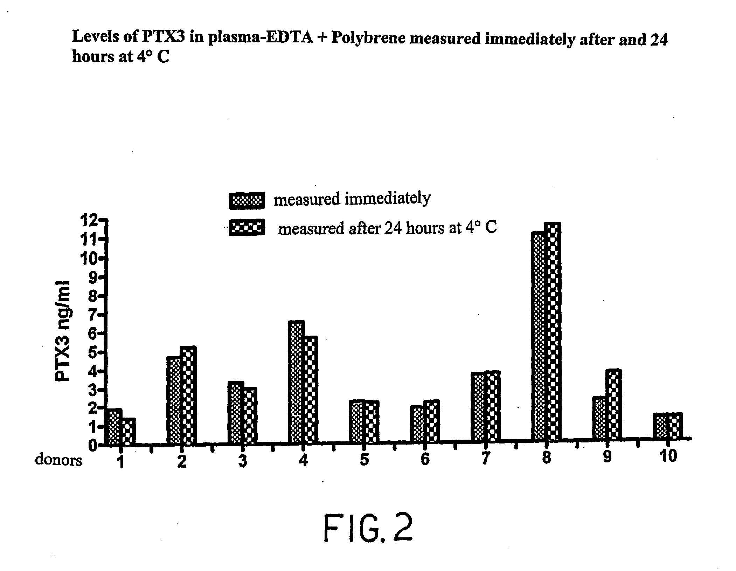

The present invention is based on the observation that the addition of a cationic polymer, particularly the polymer known by the brand name polybrene®, to EDTA-plasma allows the determination of higher PTX3 levels with respect to the PTX3 levels observed in the same EDTA-plasma samples without the addition of polybrene®, as shown in figure 1. Furthermore, the histogram in figure 2 clearly shows that the PTX3 values obtained from the sample thawed out immediately or after 24 hours at 4°C after thawing show no significant differences, as opposed to that observed previously in samples of EDTA-plasma without the addition of the cationic polymer.

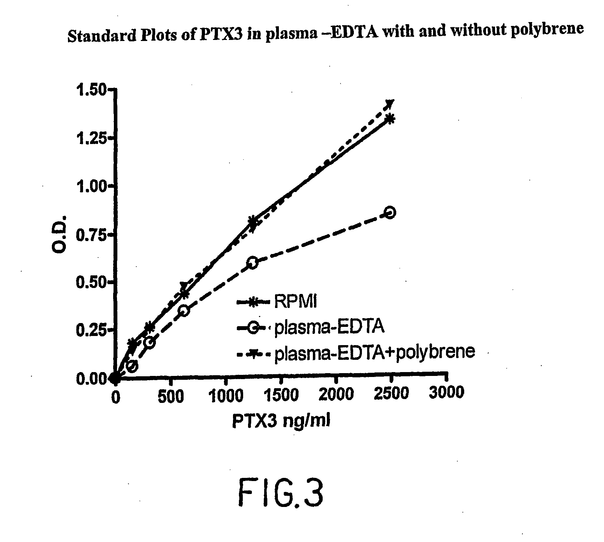

In the light of the above observations, further experiments have been performed in order to demonstrate the reliability of PTX3 measurements using the method of the invention. Indeed, Figure 3 shows a graph reporting optical density (O.D.) values plotted against plasma PTX3 levels in a standard solution of PTX3 in RPMI + 2% BSA, in EDTA-plasma without polybrene® and in EDTA-plasma with polybrene®. As may be seen from the graph, the curve of the EDTA-plasma sample with polybrene®, spiked with a standard quantity of PTX3, is practically overlappable with that of the PTX3 standard solution, while the sample of EDTA-plasma without any polybrene®, again supplemented with the same amount of PTX3 standard, shows an entirely different trend. This experiment proves that the addition of the cationic polymer to the EDTA-plasma sample allows the measurement of all the PTX3 present in the sample and hence the correct levels of the protein. Vice versa, the determination of the levels of PTX3 present in a normal EDTA-plasma sample is downward distorted, with the consequent risk of measuring false negatives in blood samples which vice versa contain high levels of PTX3.

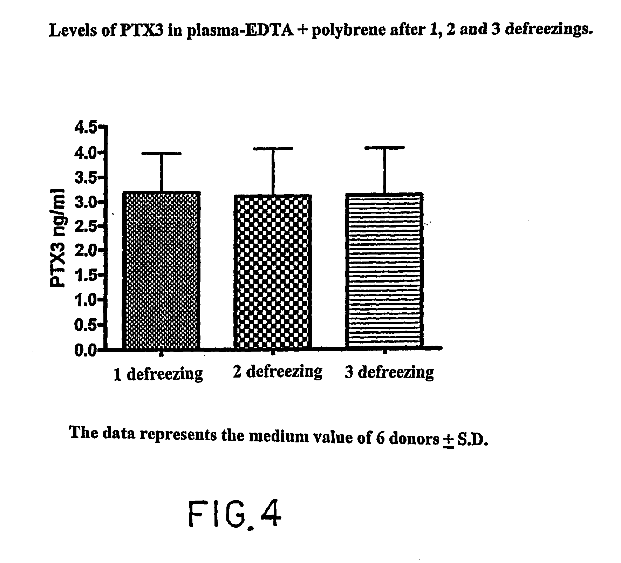

Consequently, figure 4 shows a histogram representing PTX3 levels determined on the same EDTA-plasma sample supplemented with polybrene® after 1, 2 and 3 freeze-thawing cycles, respectively. It may be observed that PTX3 levels are unaltered in the three cases cited, thanks to the fact that most likely the molecules of PTX3 are not subject to any sequestering interactions in the plasma.

Without being bound to any particular theory, it is possible that the effect observed is due to the fact that the cationic polymer disrupts interactions between the PTX3 molecules and any plasma constituents, for example red blood cells or blood proteins such as c1q protein, and that thus PTX3, free from any such interactions, can be measured completely. Again, with regard to theoretical possibilities, it is possible to hypothesise that the observed effect has some correlation with the nonspecific and reversible red blood cell agglutination activity of the cationic polymer.

Hence, an object of the present invention is a method for measuring PTX3 levels in a human or animal derived plasma sample, comprising a stage of the treatment of said plasma sample with a red blood cell agglutinating agent.

Preferably, said plasma sample is human plasma.

Preferably, said agglutinating agent is a cationic medium, more preferably a quaternary ammonium salt. In one particularly preferred embodiment of the invention, said agglutinating agent is a cationic polymer comprising one or more quaternary ammonium groups. Even more preferably, said cationic polymer is 1,5-dimethyl-1,5-diazaundecamethylene polymethobromide, also known by the name hexadimethrine bromide and sold by Sigma-Aldrich under the brand name polybrene®.

In accordance with the method of the invention, plasma, treated with an anticoagulant having a Ca⁺⁺- chelating effect, preferably EDTA (ethylene diamine tetraacetic acid) or salts of citric acid, such as sodium citrate, and then centrifuged, from hereinafter referred to as "chelating agent-plasma", are supplemented with the agglutinating agent up to a final concentration of the latter comprised of between 0.01 and 0.3% by weight with respect to the weight of the sample. Preferably, the final concentration of the agglutinating agent is comprised of between 0.02 and 0.1% by weight, more preferably, around 0.5% by weight. Said concentration may be achieved by adding between 4 and 120 µl, preferably between 8 and 40 µl, more preferably around 20 µl, of a 2.5% solution of agglutinating agent in Ca++ and Mg++ free PBS, for each ml of chelating agent-plasma.

Preferably, the chelating agent-plasma samples treated with the agglutinating agent are left standing at room temperature for 10-20 minutes, more preferably around 15 minutes, prior to determination of the levels of PTX3.

The chelating agent-plasma sample treated with the agglutinating agent of the invention will then be subjected to standard immunoassay techniques. A typical immunoassay according to the invention comprises the stages of i) exposing the treated chelating agent-plasma sample to a monoclonal or polyclonal antibody, preferably a monoclonal antibody, against PTX3, and ii) determining specific antigen/antibody binding.

Antigen/antibody binding may be measured using radioisotopic or non-radioisotopic methods. For example, these include IRMA (Immune Radioimmunometric Assay), EIA (Enzyme Immuno Assay), ELISA (Enzyme Linked Immuno Assay), FIA (Fluorescent Immuno Assay) and CLIA (Chemiluminescent Immune Assay) techniques. The method envisages the use of means of detection such as radionuclide labels, enzymes, coenzymes, fluorophores, chemiluminescence agents, chromogens, substrates, enzyme cofactors or inhibitors, the production of free-radicals, dyes etc..

In a preferred embodiment, the immunoassay is an ELISA-type assay and envisages use of the rat monoclonal antibody MNB4 (IgG2a), capable of binding specifically to PTX3 with a sensitivity of less than 100 pg/ml, and a rabbit anti-PTX3 polyclonal antibody (biotinylated IgG).

The following example describes a typical method for measuring PTX3 in a human plasma sample according to an ELISA assay. The plasma sample used is a chelating agent-plasma sample treated with 20 µl of a 2.5% solution of polybrene® in Ca++ and Mg++ free PBS per ml of plasma, so as to give a final polybrene® concentration in the sample of 0.05% by weight.

### Example

96 well ELISA plates are coated with 100 µl of MNB4 monoclonal antibody at a concentration of 700 ng/ml in 15 mM carbonate buffer at pH 9.6 and are then incubated overnight at 4°C. The monoclonal antibody has been obtained by the fusion of spleen cells from a mouse immunised with recombinant PTX3 with the murine myeloma cell line SP2/O.

The plates are then washed three times (300 µl/well) with a wash buffer solution (PBS + 0.005% Tween20) and subsequently, 300 µl of wash buffer with 5% dried milk powder are added to each well in order to block nonspecific binding sites.

After incubating for 2 hours at room temperature, the plates are washed three times with 300 µl of wash buffer.

Into each well, in duplicate, are added 50 µl of recombinant human PTX3 standard (two-fold serial dilutions between 1.2 ng/ml and 75 pg/ml) and EDTA-plasma samples, treated as indicated above, diluted two-fold in PBS + 2% BSA added to the other wells, and the plates incubated for two hours at 37°C.

The plates are then washed five times with wash buffer, and then 100 µl of rabbit anti-PTX3 biotinylated IgG (25 ng/ml) in wash buffer are added to each well.

After incubating for 1 hour at 37°C, the plates are washed five times with 300 µl of wash buffer.

100 µl of Amdex streptavidin horseradish peroxidase conjugate (RPN 4401 Amersham), diluted 1 to 8000, are added to each well, and the plates incubated at room temperature for 1 hour.

After having washed the plates five times with wash buffer, 100 µl of TMB (tetramethylbenzidine) are added to each well and the plates incubated at room temperature for 5 minutes. The enzyme reaction is terminated by the addition of 50 µl of a solution of 1M H2SO4 and the absorbance is then read at 450nm within 30 minutes of the reaction being terminated.

A further object of the present invention is a method for determining the prognosis for cardiovascular and cerebrovascular disorders comprising a stage of measuring the levels of PTX3 in a human or animal, preferably human, plasma sample according to the previously described method and a stage of comparing PTX3 plasma levels thus determined with a reference value, wherein plasma levels of PTX3 higher than said reference value are an indicator of increased risk of mortality or cardiac decompensation, after myocardial infarction, or death or complications, following cerebral stroke.

In particular, said PTX3 reference value is equal to the level of plasma PTX3 that can be determined in healthy individuals and is preferably equal to 2 ng/ml, more preferably 5 ng/ml PTX3.

More preferably, said plasma sample will be a sample deriving from a patient having undergone a myocardial infarction 8-12 hours prior to collection of the sample, or a cerebral stroke approx. 24 hours prior to collection.

A kit comprising anti-PTX3 monoclonal and polyclonal antibodies and a red blood cell agglutinating agent, in predetermined quantities and concentrations, constitutes a further object of the invention.

Preferably, said antibodies will be a rat monoclonal antibody MNB4 (IgG2a) and a rabbit polyclonal antibody (biotinylated IgG).

Preferably, said agglutinating agent will be selected from those defined above, particularly 1,5-dimethyl-1,5-diazaundecamethylene polymethobromide.

Optionally, the kit of the invention may comprise recombinant human PTX3, detection reagents, buffers, diluents, stabilisers and other reagents of use in performing the immunoassay, in joined or separate containers.

The method of the invention has the advantage of giving a precise and reliable measurement of PTX3 in a plasma sample, avoiding false negatives.

The method of the invention further allows the stabilisation of the PTX3 in the plasma sample under test, thus giving precise levels even after repeated freeze-thawing cycles.

A further advantage of the invention is that, contrary to the previous method, the assay method claimed allows determining the plasma PTX3 level equally well in EDTA-plasma or citrate-plasma samples, with the same degree of reliability.

## Claims

1. A method for measuring PTX3 levels in a human or animal derived plasma sample, comprising a stage of the treatment of said plasma sample with a red blood cell agglutinating agent and a subsequent stage of determining the plasma levels of PTX3.

2. The method according to claim 1, wherein said red blood cell agglutinating agent is a cationic medium.

3. The method according to claim 2, wherein said cationic medium is a quaternary ammonium salt.

4. The method according to any of the claims 1 to 3, wherein said red blood cell agglutinating agent is a cationic polymer containing one or more quaternary ammonium groups.

5. The method according to claim 4, wherein said cationic polymer is 1,5-dimethyl-1,5-diazaundecamethylene polymethobromide.

6. The method according to any of the claims 1 to 5, wherein said plasma is plasma treated with a Ca⁺⁺- chelating anticoagulant.

7. The method according to claim 5, wherein said Ca⁺⁺-chelating anticoagulant is selected from EDTA and a salt of citric acid.

8. The method according to any of the claims 1 to 7, wherein said red blood cell agglutinating agent is added to the plasma up to a final concentration comprised of between 0.01 and 0.3% by weight, with respect to the weight of the sample.

9. The method according to claim 8, wherein said final concentration of agglutinating agent is comprised of between 0.02 and 0.1% by weight, preferably approx. 0.5% by weight, with respect to the weight of the sample.

10. The method according to claim 8, wherein said agglutinating agent, as a 2.5% solution in Ca⁺⁺ and Mg⁺⁺ free PBS, is added in quantities comprised of between 4 and 120 µl for each ml of plasma.

11. The method according to claim 9, wherein said agglutinating agent, as a 2.5% solution in Ca++ and Mg++ free PBS, is added in quantities comprised of between 8 and 40 µl, preferably approx. 20 µl, for each ml of plasma.

12. The method according to any of the claims 1 to 11, wherein said plasma sample treated with said red blood cell agglutinating agent is allowed to stand at room temperature for 10-20 minutes, preferably approx. 15 minutes, prior to said stage of determining plasma levels of PTX3.

13. The method according to any of the claims 1 to 12, wherein said stage of determining plasma levels of PTX3 is carried out by means of an immunoassay and comprises the stages of: i) exposing the plasma sample to a monoclonal and/or polyclonal antibody against PTX3, and ii) determining specific antigen/antibody binding.

14. The method according to claim 13, wherein said determination of specific antigen/antibody binding is performed by means of a method selected from IRMA (Immune Radioimmunometric Assay), EIA (Enzyme Immuno Assay), ELISA (Enzyme Linked Immuno Assay), FIA (Fluorescent Immuno Assay), CLIA (Chemiluminescent Immune Assay) .

15. The method according to claim 14, wherein said immunoassay is an ELISA-type assay using the rat monoclonal antibody MNB4 (IgG2a) and an anti-PTX3 rabbit polyclonal antibody (biotinylated IgG).

16. A method for determining the prognosis for cardiovascular and cerebrovascular disorders, comprising a stage of measuring the levels of PTX3 in a human or animal plasma sample according to the method described in any of the claims 1 to 15, and a stage of comparing the PTX3 plasma levels thus determined with a reference value, wherein plasma levels of PTX3 higher than said reference value are an indicator of increased risk of mortality or cardiac decompensation, after myocardial infarction, or mortality or complications, following cerebral stroke.

17. The method according to claim 16, wherein said PTX3 reference value is equal to the plasma level of PTX3 that can be determined in healthy individuals.

18. The method according to claims 16 or 17, wherein said PTX3 reference value is equal to 2 ng/ml, preferably 5 ng/ml PTX3.

19. The method according to any of the claims 16 to 18, wherein said plasma sample is from a patient who has undergone a myocardial infarction 8-12 hours prior to collection of the sample, or a cerebral stroke approx. 24 hours prior to collection of the sample.

20. A kit for determining plasma levels of PTX3 in a plasma sample, comprising anti-PTX3 monoclonal and/or polyclonal antibodies and a red blood cell agglutinating agent in predetermined quantities and concentrations.

21. The kit according to claim 20, wherein said antibodies are a rat monoclonal antibody MNB4 (IgG2a) and a rabbit polyclonal antibody (biotinylated IgG).

22. The kit according to claims 20 or 21, wherein said red blood cell agglutinating agent is as defined in any of the claims 2 to 5.

23. The kit according to any of the claims 20 to 22, comprising one or more components selected from recombinant human PTX3, detection reagents, buffers, diluents, stabilisers in joined or separate containers.

## Patentansprüche

1. Verfahren zum Messen von PTX3-Level in einer Plasmaprobe, die aus einem Menschen oder Tier stammt, umfassend eine Phase der Behandlung der Plasmaprobe mit einem Agglutinierungsmittel für rote Blutkörperchen und einer darauf folgenden Phase des Bestimmens der Plasmalevel von PTX3.

2. Verfahren nach Anspruch 1, wobei das Agglutinierungsmittel für rote Blutkörperchen ein kationisches Medium ist.

3. Verfahren nach Anspruch 2, wobei das kationische Medium ein quartäres Ammoniumsalz ist.

4. Verfahren nach einem der Ansprüche 1 bis 3, wobei das Agglutinierungsmittel für rote Blutkörperchen ein kationisches Polymer ist, das eine oder mehr quartäre Ammoniumgruppen enthält.

5. Verfahren nach Anspruch 4, wobei das kationische Polymer 1,5-Dimethyl-1,5-Diazaundecamethylen-polymethobromid ist.

6. Verfahren nach einem der Ansprüche 1 bis 5, wobei das Plasma Plasma ist, das mit einem Ca⁺⁺-chelatisiernden Antikoagulanz behandelt wird.

7. Verfahren nach Anspruch 5, wobei das Ca⁺⁺-chelatisierende Antikoagulanz ausgewählt ist aus EDTA und einem Salz der Zitronensäure.

8. Verfahren nach einem der Ansprüche 1 bis 7, wobei das Agglutinierungsmittel für rote Blutkörperchen zum Plasma bis zu einer Endkonzentration, die zwischen 0,01 und 0,3 Gewichts-% umfasst, bezogen auf das Gewicht der Probe, gegeben wird.

9. Verfahren nach Anspruch 8, wobei die Endkonzentration des Agglutinierungsmittels zwischen 0,02 und 0,1 Gewichts-% umfasst, vorzugsweise ungef. 0,5 Gewichts-%, bezogen auf das Gewicht der Probe.

10. Verfahren nach Anspruch 8, wobei das Agglutinierungsmittel als eine 2,5 %-Lösung in Ca⁺⁺- und Mg⁺⁺-freiem PBS, in Mengen, die zwischen 4 und 120 µl für jeden ml Plasma umfassen, zugegeben wird.

11. Verfahren nach Anspruch 9, wobei das Agglutinierungsmittel als eine 2,5 %-Lösung in Ca⁺⁺- und Mg⁺⁺-freiem PBS, in Mengen, die zwischen 8 und 40 µl, vorzugsweise ungef. 20 µl für jeden ml Plasma umfassen, zugegeben wird.

12. Verfahren nach einem der Ansprüche 1 bis 11, wobei die Plasmaprobe, die mit dem Agglutinierungsmittel für rote Blutkörperchen behandelt wurde, bei Raumtemperatur für 10-20 Minuten stehen gelassen wird, vorzugsweise ungef. 15 Minuten, vor der Phase des Bestimmens der Plasmalevel von PTX3.

13. Verfahren nach einem der Ansprüche 1 bis 12, wobei die Phase des Bestimmens der Plasmalevel von PTX3 durch einen Immunoassay durchgeführt wird und die Schritte: i) Aussetzen der Plasmaprobe einem monoklonalen und/oder polyklonalen Antikörper gegen PTX3 und ii) Bestimmen spezifischer Antigen/Antikörper-Bindung, umfasst.

14. Verfahren nach Anspruch 13, wobei die Bestimmung der spezifischen Antigen/Antikörper-Bindung durch ein Verfahren ausgewählt aus IRMA (immunradiometrischer Assay), EIA (Enzym-Immunoassay), ELISA (Enzymgekoppelter Immunadsorptionsassay), FIA (Fluoreszenz-Immunoassay), CLIA (Chemilumineszenz-Immunoassay) durchgeführt wird.

15. Verfahren nach Anspruch 14, wobei der Immunoassay ein Assay vom ELISA-Typ unter Verwendung des monoklonalen Ratten-Antikörpers MNB4 (lgG2a) und eines polyklonalen anti-PTX3 Kaninchen-Antikörpers (biotinyliertes IgG) ist.

16. Verfahren zum Bestimmen der Prognose für kardiovaskuläre und zerebrovaskuläre Störungen, umfassend eine Phase des Messens der Level von PTX3 in einer Menschen- oder Tier-Plasmaprobe gemäß dem Verfahren, das in einem der Ansprüche 1 bis 15 beschrieben wurde, und eine Phase des Vergleichens der PTX3-Plasmalevel, die folglich mit einem Bezugswert bestimmt werden, wobei Plasmalevel von PTX3, die höher als der Bezugswert sind, ein Indikator für ein erhöhtes Risiko der Mortalität oder Herzdekompensation nach Myokardinfarkt sind, oder Mortalität oder Komplikationen nach Hirnschlag.

17. Verfahren nach Anspruch 16, wobei der PTX3-Bezugswert dem Plasmalevel von PTX3, der in gesunden Individuen bestimmt werden kann, gleich ist.

18. Verfahren nach Anspruch 16 oder 17, wobei der PTX3-Bezugswert gleich 2 ng/ml, vorzugsweise 5 ng/ml PTX3 ist.

19. Verfahren nach einem der Ansprüche 16 bis 18, wobei die Plasmaprobe aus einem Patienten ist, der einen Myokardinfarkt 8-12 Stunden vor dem Abnehmen der Probe hatte oder einen Hirnschlag ungef. 24 Stunden vor dem Abnehmen der Probe.

20. Kit zum Bestimmen der Plasmalevel von PTX3 in einer Plasmaprobe, umfassend monoklonalen und/oder polyklonalen anti-PTX3 Antikörper und ein Agglutinierungsmittel für rote Blutkörperchen in vorbestimmten Mengen und Konzentrationen.

21. Kit nach Anspruch 20, wobei die Antikörper ein monoklonaler Ratte-Antikörper MNB4 (lgG2a) und ein polyklonaler Kaninchen-Antikörper (biotinyliertes IgG) sind.

22. Kit nach Anspruch 20 oder 21, wobei das Agglutinierungsmittel für rote Blutkörperchen wie in einem der Ansprüche 2 bis 5 definiert, ist.

23. Kit nach einem der Ansprüche 20 bis 22, umfassend einen oder mehrere Bestandteil(e) ausgewählt aus rekombinantem humanem PTX3, Detektionsreagenzien, Puffern, Verdünnungsmitteln, Stabilisierern in verbundenen oder getrennten Behältern.

## Revendications

1. Procédé de mesure des taux de PTX3 dans un échantillon plasmatique d'origine humaine ou animale, comprenant un stade de traitement dudit échantillon plasmatique par un agent agglutinant des érythrocytes et un stade subséquent de détermination des taux plasmatiques de PTX3.

2. Procédé selon la revendication 1, où ledit agent agglutinant les érythrocytes est un milieu cationique.

3. Procédé selon la revendication 2, où ledit milieu cationique est un sel d'ammonium quaternaire.

4. Procédé selon l'une quelconque des revendications 1 à 3, où ledit agent agglutinant les érythrocytes est un polymère cationique contenant un ou plusieurs groupes ammonium quaternaire.

5. Procédé selon la revendication 4, où ledit polymère cationique est du polyméthobromure de 1,5-diméthyl-1,5-diazaundécaméthylène.

6. Procédé selon l'une quelconque des revendications 1 à 5, où ledit plasma est un plasma traité par un anticoagulant chélatant Ca⁺⁺.

7. Procédé selon la revendication 5, où ledit anticoagulant chélatant Ca⁺⁺ est sélectionné parmi l'EDTA et un sel d'acide citrique.

8. Procédé selon l'une quelconque des revendications 1 à 7, où ledit agent agglutinant les érythrocytes est ajouté au plasma jusqu'à une concentration finale comprise entre 0,01 et 0,3 % en poids, par rapport au poids de l'échantillon.

9. Procédé selon la revendication 8, où ladite concentration finale d'agent agglutinant est comprise entre 0,02 et 0,1 % en poids, est de préférence d'environ 0,5 % en poids, par rapport au poids de l'échantillon.

10. Procédé selon la revendication 8, où ledit agent agglutinant, sous forme de solution à 2,5 % dans du PBS exempt de Ca⁺⁺ et Mg⁺⁺, est ajouté en quantités comprises entre 4 et 120 µl par ml de plasma.

11. Procédé selon la revendication 9, où ledit agent agglutinant, sous forme de solution à 2,5 % dans du PBS exempt de Ca⁺⁺ et Mg⁺⁺, est ajouté en quantités comprises entre 8 et 40 µl, de préférence d'environ 20 µl par ml de plasma.

12. Procédé selon l'une quelconque des revendications 1 à 11, où on laisse reposer ledit échantillon de plasma traité par ledit agent agglutinant les érythrocytes à température ambiante pendant 10 à 20 min, de préférence pendant environ 15 min, avant ledit stade de détermination des taux plasmatiques de PTX3.

13. Procédé selon l'une quelconque des revendications 1 à 12, où ledit stade de détermination des taux plasmatiques de PTX3 est mis en oeuvre au moyen d'un dosage immunologique et comprend les stades de : i) exposition de l'échantillon plasmatique à un anticorps monoclonal et/ou polyclonal dirigé contre PTX3 et ii) détermination de la liaison spécifique antigène/anticorps.

14. Procédé selon la revendication 13, où ladite détermination de la liaison spécifique antigène/anticorps est réalisée au moyen d'un procédé sélectionné parmi IRMA (dosage immunoradioimmunométrique), EIA (dosage immunoenzymatique), ELISA (dosage immunoenzymatique utilisant un antigène adsorbé), FIA (dosage immunofluorescent), CLIA (dosage immunochimioluminescent).

15. Procédé selon la revendication 14, où ledit dosage immunologique est un dosage de type ELISA utilisant l'anticorps monoclonal de rat MNB4 (IgG2a) et un anticorps polyclonal de lapin anti-PTX3 (IgG biotinylée).

16. Procédé de détermination du pronostic de troubles cardio-vasculaires et cérébrovasculaires, comprenant un stade de mesure des taux de PTX3 dans un échantillon plasmatique humain ou animal selon le procédé selon l'une quelconque des revendications 1 à 15 et un stade de comparaison des taux plasmatiques de PTX3 ainsi déterminés avec une valeur de référence, où les taux plasmatiques de PTX3 supérieurs à ladite valeur de référence indiquent un risque accru de mortalité ou de décompensation cardiaque après un infarctus myocardique ou de mortalité ou de complications après un accident vasculaire cérébral.

17. Procédé selon la revendication 16, où ladite valeur de référence de PTX3 est égale au taux plasmatique de PTX3 susceptible d'être déterminé chez des individus sains.

18. Procédé selon la revendication 16 ou 17, où ladite valeur de référence de PTX3 est égale à 2 ng/ml, de préférence 5 ng/ml de PTX3.

19. Procédé selon l'une quelconque des revendications 16 à 18, où ledit échantillon plasmatique provient d'un patient ayant fait un infarctus myocardique 8 à 12 h avant le recueil de l'échantillon ou un accident vasculaire cérébral environ 24 h avant le recueil de l'échantillon.

20. Kit de détermination des taux plasmatiques de PTX3 dans un échantillon de plasma, comprenant des anticorps monoclonaux et/ou polyclonaux anti-PTX3 et un agent agglutinant les érythrocytes en des quantités et concentrations prédéterminées.

21. Kit selon la revendication 20, où lesdits anticorps sont un anticorps monoclonal de rat MNB4 (IgG2a) et un anticorps polyclonal de lapin (IgG biotinylée).

22. Kit selon la revendication 20 ou 21, où ledit agent agglutinant les érythrocytes est tel que défini selon l'une quelconque des revendications 2 à 5.

23. Kit selon l'une quelconque des revendications 20 à 22, comprenant un ou plusieurs composants sélectionnés parmi une PTX3 humaine recombinée, des réactifs de détection, des tampons, des diluants, des stabilisants dans des récipients réunis ou distincts.
